# FASCICULE DE BREVET EUROPEEN

(11) **EP 1 045 670 B1**
(45) Date de publication et mention de la délivrance du brevet: **10.01.2007**
(21) Numéro de dépôt: 99971687.1
(22) Date de dépôt: 10.11.1999
(51) Int. Cl.: A61B 17/02

(54) **DISPOSITIF D'ECARTEMENT POUR ACCEDER PAR VOIE ANTERIEURE A UNE PARTIE DU RACHIS**
DISTRAKTIONSVORRICHTUNG ZUM ÄUSSEREN ZUGRIFF AUF EINEN TEIL DER WIRBELSAÜLE
SPACING DEVICE FOR ACCEDING BY ANTERIOR APPROACH TO A PART OF THE SPINE

(30) Priorité: 10.11.1998 FR 9814128
(43) Date de publication de la demande: 25.10.2000
(73) Titulaire: Euros, 13600 La Ciotat (FR)
(72) Inventeur: ONIMUS, Michel, F-25000 Besançon (FR)
(74) Mandataire: Somnier, Jean-Louis
(86) Numéro de dépôt international: PCT/FR1999/002766
(87) Numéro de publication internationale: WO 2000/027291

(56) Documents cités:
- EP-A- 0 455 282
- EP-A- 0 749 724
- WO-A-98/27869
- FR-A- 2 662 929
- FR-A- 2 770 124
- US-A- 5 125 396

## Description

La présente invention concerne un dispositif d'écartement de parties molles d'un corps humain ou d'un vertébré pour accéder par voie antérieure à une partie du rachis dudit corps.

Les interventions chirurgicales sur le rachis peuvent être effectuées par voie antérieure ou postérieure.

Les interventions chirurgicales sur le rachis par voie antérieure, qui nous intéresse ici plus particulièrement, sont utilisées pour traiter différentes pathologies rachidiennes telles que les déformations et instabilités rachidiennes, les tumeurs et traumatismes, et notamment pour aborder le rachis lombaire afin d'opérer un disque intervertébral particulier.

L'intervention chirurgicale par voie antérieure consiste à pratiquer une petite incision médiane dans le ventre du patient et à écarter les parties molles du patient pour accéder à une partie du rachis.

Une telle intervention nécessite alors l'utilisation de dispositifs ou appareils particuliers pour maintenir écartées durant l'intervention, les parties molles du patient telles que le foie, l'abdomen, l'intestin etc, afin de pouvoir accéder à la partie du rachis à opérer.

Les dispositifs de ce type actuellement connus présentent comme inconvénients principaux d'être très onéreux. Ils sont en outre encombrants et difficiles à manipuler.

En particulier, on connaît déjà un dispositif de ce type qui comporte un cadre rectangulaire extérieur sur lequel sont montées quatre tiges filetées disposées en étoile, s'étendant vers l'intérieur du cadre et pourvues à une de leurs extrémités d'une paroi d'écartement destinée à être introduite le corps du patient incisé pour écarter les parties molles de ce dernier.

Il comporte en outre des molettes vissées sur les tiges filetées à l'extérieur du cadre, à l'aide desquelles on déplace lesdites tiges filetées pour plaquer les parois d'écartement sur les parties molles et retenir lesdites parties molles ainsi écartées du rachis durant l'intervention.

Un tel dispositif est bien entendu très encombrant, relativement difficile à manipuler, et onéreux.

Le document EP-A-0 749 724 décrit un dispositif d'écartement comprenant deux parois d'écartement comprenant des moyens de fixation sur une vertèbre, ainsi que des moyens de solidarisation de ces parois entre elles. Les parois d'écartement ne sont pas pourvues d'un panneau fixe et d'un panneau mobile coulissant.

Le document FR-A-2 662 929 montre une paroi comprenant un panneau fixe et deux panneaux coulissants. Aucun moyen de fixation sur une vertèbre n'est cependant décrit.

Afin de remédier aux inconvénients de l'état de la technique précité, la présente invention propose un dispositif d'écartement de parties molles d'un corps humain ou d'un vertébré pour accéder par voie antérieure à une partie du rachis dudit corps, caractérisé en ce qu'il comprend :
- une première et une deuxième parois d'écartement de parties molles, disposées en regard l'une de l'autre, et pourvues chacune d'un panneau fixe et d'un panneau mobile apte à coulisser par rapport audit panneau fixe, sensiblement transversalement à la direction longitudinale desdites parois d'écartement, ces dernières étant destinées à être positionnées chacune sensiblement verticalement, à cheval sur deux vertèbres adjacentes du rachis,
- des moyens de fixation de chaque panneau fixe sur une vertèbre et de chaque panneau mobile à l'autre vertèbre adjacente, et
- des moyens de solidarisation des panneaux fixes entre eux.

Un tel dispositif est avantageusement peu encombrant donc convivial à utiliser.

De plus, sa fabrication s'avère d'un coût relativement faible.

D'autres caractéristiques non limitatives et avantageuses du dispositif d'écartement selon l'invention sont les suivantes :
- il comprend une troisième paroi d'écartement de parties molles, comportant un panneau fixe positionné dans un plan longitudinal et perpendiculaire aux plans desdites première et deuxième parois, et des moyens de montage de la troisième paroi d'écartement auxdits moyens de solidarisation, ladite troisième paroi étant destinée à être positionnée de manière fixe, sensiblement verticalement au niveau d'une des deux vertèbres supportant les première et deuxième parois d'écartement, de sorte qu'elle fait face aux deux bords longitudinaux externes des panneaux fixes en regard desdites première et deuxième parois d'écartement,
- il comporte une poignée de préhension et de manoeuvre montée de manière amovible sur chaque paroi d'écartement,
- les première et deuxième parois d'écartement présentent un profil courbe et sont placées de sorte que leur face incurvée soit tournée vers les vertèbres, le coulissement des panneaux mobiles par rapport aux panneaux fixes s'effectuant suivant un arc de cercle,
- les première et deuxième parois d'écartement sont symétriques par rapport à un axe vertical passant entre les deux vertèbres,
- lesdits moyens de solidarisation comprennent une pièce plate en forme de C positionnée dans un plan horizontal et pourvue à chaque extrémité d'un orifice dans lequel est engagé un pion vertical solidaire d'un panneau fixe d'une des première et deuxième parois d'écartement,
- cette pièce plate en forme de C peut comporter à chaque extrémité deux orifices adjacents, ce qui permet d'ajuster la position relative des première et deuxième parois d'écartement,
- la pièce plate en forme de C peut comporter dans sa partie centrale, un logement de réception pour le montage de la troisième paroi d'écartement,
- la paroi d'écartement comporte alors en partie supérieure une pluralité de fentes horizontales, chaque fente étant apte à coopérer par engagement avec le logement de réception de ladite partie plate en forme de C.

La description qui va suivre en regard des dessins annexés, donnés à titre d'exemples non limitatifs, fera bien comprendre en quoi consiste l'invention et comment elle peut être réalisée.

Sur les dessins annexés :
- la figure 1 est une vue schématique en perspective d'un mode de réalisation du dispositif d'écartement selon l'invention,
- la figure 2 est une vue schématique de détail d'une des première et deuxième parois d'écartement du dispositif de la figure 1,
- les figures 3 et 4 sont des vues de dessus représentant deux étapes de la mise en place du dispositif de la figure 1 sur deux vertèbres adjacentes.

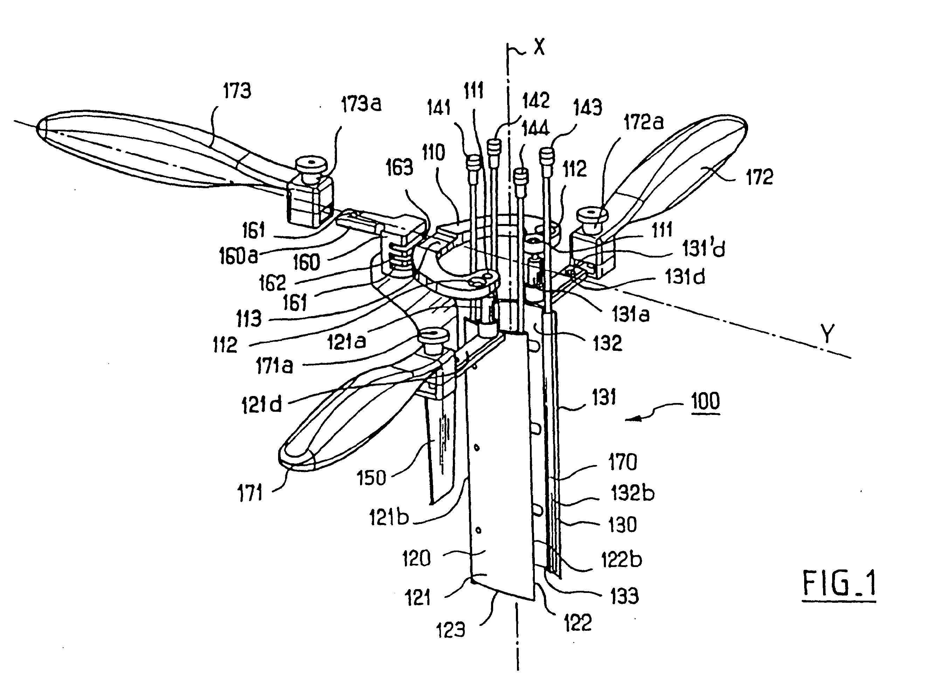

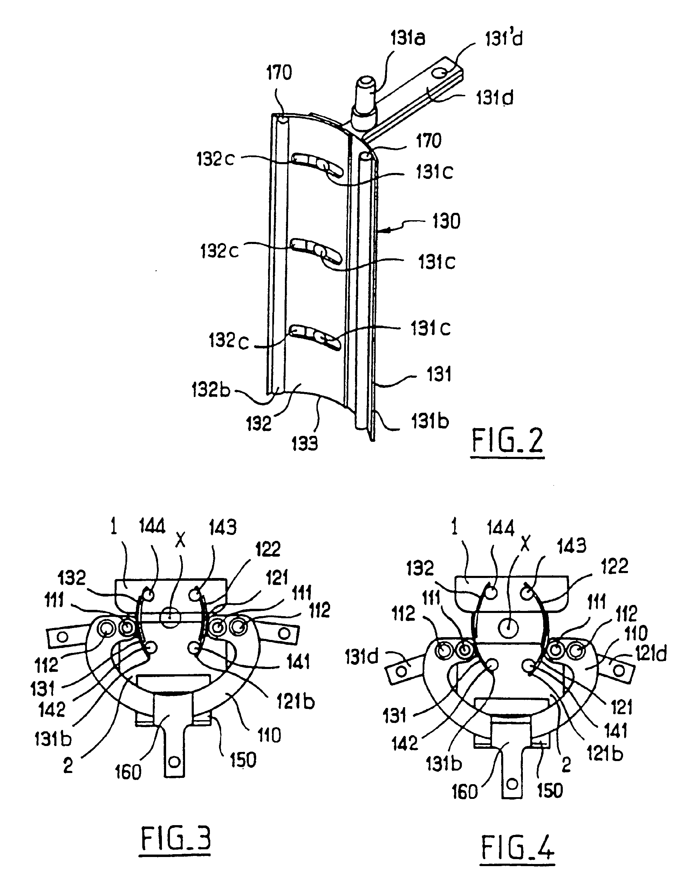

En référence aux figures 1 et 2, on a représenté un dispositif d'écartement 100 de parties molles (abdomen, intestin, foie...) d'un corps humain ou d'un vertébré, pour accéder par voie antérieure à une partie du rachis dudit corps.

Ce dispositif d'écartement 100 comprend une première et une deuxième parois d'écartement 120, 130 de parties molles, disposées en regard l'une de l'autre, et destinées à être positionnées chacune sensiblement verticalement, à cheval sur deux vertèbres adjacentes du rachis.

Chacune des première et deuxième parois d'écartement 120, 130 est pourvue d'un panneau fixe 121, 131 et d'un panneau mobile 122, 132 apte à coulisser par rapport audit panneau fixe 121, 131, sensiblement transversalement à la direction longitudinale desdites parois d'écartement 120, 130.

Lorsque lesdites parois d'écartement 120, 130 sont positionnées sur les vertèbres, le coulissement d'un panneau par rapport à l'autre s'effectue selon une direction sensiblement horizontale.

Selon l'exemple représenté, les première et deuxième parois d'écartement 120, 130 présentent un profil courbe et sont placées de sorte que leur face incurvée soit tournée vers les vertèbres, le coulissement des panneaux mobiles 122, 132 par rapport aux panneaux fixes 121, 131 s'effectuant suivant un arc de cercle.

Ici, les première et deuxième parois d'écartement 120, 130 sont symétriques par rapport à un axe vertical X passant entre les deux vertèbres adjacentes.

De plus, les première et deuxième parois d'écartement 120, 130 comportent chacune un bord inférieur, 123, 133 d'appui sensiblement en forme d'arche, de façon à s'adapter au mieux à la forme des vertèbres support et d'éviter ainsi que des parties molles restent coincées entre lesdites vertèbres et lesdites parois.

Le panneau mobile 122, 132 de chacune des première et deuxième parois d'écartement 120, 130 est positionné à l'intérieur (c'est-à-dire le plus proche du rachis) du panneau fixe 121, 131.

Chaque panneau mobile 122, 132 coulisse par rapport à chaque panneau fixe 121, 131 en glissant sur la face interne incurvée de ce dernier.

Selon l'exemple représenté, l'assemblage des panneaux fixe et mobile de chacune des première et deuxième parois d'écartement, est décrit ci-après en référence à la figure 2.

Chaque panneau mobile 132 des première et deuxième parois d'écartement est pourvu de lumières 132c allongées selon la direction transversale dudit panneau.

Ici les lumières 132c suivent la courbure dudit panneau et sont réparties sur sa hauteur.

Lesdites lumières 132c présentent par exemple une forme oblongue.

Dans ces lumières allongées 132c sont montés à coulissement des tétons 131c prévus en saillie sur la face incurvée de chaque panneau fixe 131.

Bien entendu, selon une variante non représentée, on peut prévoir que les lumières soient ménagées dans les panneaux fixes et les tétons soient portés par les panneaux mobiles des première et deuxième parois d'écartement.

On peut également prévoir tout autre mode de guidage (tel que des glissières par exemple) des panneaux fixe et mobile l'un par rapport à l'autre.

En outre, chaque bord externe longitudinal 121b, 131 b, 122b, 132b de chacun des panneaux fixes 121, 131 et mobiles 122, 132 des première et deuxième parois d'écartement 120, 130, comprend un conduit de réception 170, ici de forme tubulaire, ouvert à chaque extrémité pour le passage d'un clou de fixation 141, 142, 144, 143.

Ces clous 141, 142, 143, 144 permettent la fixation de chaque panneau fixe 121, 131 sur une vertèbre et de chaque panneau mobile 122, 132 sur l'autre vertèbre adjacente.

Les clous 141, 142, 143, 144 sont destinés à être engagés verticalement dans les conduits tubulaires de réception des panneaux fixes et mobiles et à être enfoncés dans lesdites vertèbres.

En outre, dans le dispositif d'écartement 100 selon l'invention, il est prévu des moyens de solidarisation des panneaux fixes 121, 131 entre eux pour stabiliser le dispositif d'écartement placé dans le corps du patient.

Ces moyens de solidarisation comprennent selon l'exemple préféré et représenté sur la figure 1, une pièce plate en forme de C 110 positionnée dans un plan horizontal, et pourvue à chaque extrémité d'un orifice 111 dans lequel est engagé un pion vertical 121a, 131a d'un panneau fixe 121, 131.

Les pions verticaux 121a, 131a présentent une forme généralement cylindrique et les orifices 111 de la pièce plate en forme de C sont de forme circulaire dont le diamètre est légèrement supérieur au diamètre externe des pions verticaux de manière à autoriser un léger pivotement des parois 120, 130 autour d'un axe vertical parallèle à l'axe X permettant d'ajuster le positionnement angulaire relatif desdites parois d'écartement 120, 130 lors de leur mise en place sur les vertèbres du patient. La dimension des orifices 111 de la pièce 110 autorise également un léger pivotement des parois 120, 130 autour d'un axe horizontal parallèle à l'axe Y.

En outre, avantageusement, comme cela est représenté sur la figure 1, il est prévu dans chaque extrémité de la pièce plate en forme de C 110 un deuxième orifice 112 adjacent au première orifice 111, dans lequel peut être également engagé le pion vertical 121a, 131a de chaque panneau fixe 121, 131.

Ce deuxième orifice 112 sensiblement identique à l'orifice 111, permet également d'ajuster la position relative (l'écartement) des première et deuxième parois d'écartement 120, 130 lors de leur mise en place sur les vertèbres du patient.

Par ailleurs, dans le dispositif d'écartement 100, il est prévu une troisième paroi d'écartement des parties molles comportant un panneau fixe 150 positionné dans un plan longitudinal (vertical) et perpendiculaire aux plans verticaux des première et deuxième parois 120, 130, et des moyens de montage 160 de la troisième paroi d'écartement 150 auxdits moyens de solidarisation 110.

Cette troisième paroi d'écartement 150 est destinée à être positionnée de manière rigide, sensiblement verticalement au niveau de la vertèbre sur laquelle sont fixés les panneaux fixes 121, 131 des première et deuxième parois d'écartement 120, 130 de sorte qu'elle fait face aux bords longitudinaux externes 121 b, 131b des panneaux fixes en regard 121, 131 desdites première et deuxième parois d'écartement 120, 130.

Cette troisième paroi d'écartement 150 a une épaisseur telle (ici de l'ordre du millimètre) qu'elle présente une certaine flexibilité.

Selon l'exemple particulier représenté sur les figures, la pièce plate en forme de C 110 comporte dans sa partie centrale un logement de réception 113 pour le montage de la troisième paroi d'écartement 150.

A cet effet, la troisième paroi d'écartement 150 comporte en partie supérieure une pluralité de fentes horizontales 161, 162, 163, chaque fente étant apte à coopérer par engagement avec le logement de réception 113 de la pièce plate en forme de C 110.

Les fentes 161, 162, 163 sont superposées de façon à permettre un ajustement en hauteur de la troisième paroi d'écartement de parties molles lors de sa mise en place dans le corps incisé du patient.

En outre, comme le montre plus particulièrement la figure 1, le dispositif d'écartement comporte avantageusement une poignée de préhension et de manoeuvre 171, 172, 173 montée de manière amovible sur chaque paroi d'écartement 120, 130, 150 de parties molles.

Les panneaux fixes 121, 131 des première et deuxième parois d'écartement 120, 130, comportent en partie supérieure des barrettes de montage 121d, 131d des poignées amovibles, qui s'étendent horizontalement vers l'extérieur du dispositif et supportent à proximité desdits panneaux fixes, les pions verticaux 121a, 131a pour le montage dans la pièce plate en forme de C 110.

Ces barrettes horizontales 121,d, 131d comportent à leur extrémité externe des orifices 131'd ou autre aménagement pour le montage par encliquetage ou par vissage des poignées amovibles de manoeuvre 171, 172 comportant elles-mêmes à cet effet, un dispositif d'encliquetage ou de vissage 171a, 172a.

La troisième paroi d'écartement 150 comprend au niveau de son dispositif de montage 160 ou autre aménagement, une barrette 160a s'étendant horizontalement vers l'extérieur dudit dispositif. Cette barrette 160a est pourvue à son extrémité extérieure d'un orifice 161 pour le montage par encliquetage ou par vissage de la poignée amovible 173 à l'aide d'un dispositif de vissage ou d'encliquetage 173a.

Les trois parois d'écartement du dispositif d'écartement 100 selon l'invention peuvent présenter des longueurs différentes par exemple 100 mm, 130 mm et 160 mm.

Les clous de fixation 141, 142, 143, 144 des première et deuxième parois d'écartement 120, 130 présentent alors une longueur légèrement supérieure à celle des parois pour être enfoncés dans les vertèbres support.

Le dispositif d'écartement 100 est réalisé avantageusement en alliage métallique médical, ou en matériau composite radiotransparent ou non.

Bien entendu, on peut envisager tout autre matériau médical adapté.

Le procédé de mise en place d'un tel dispositif d'écartement 100 va être décrit ci-après en référence aux figures 3 et 4.

Dans une première étape, après avoir incisé le corps du patient pour ménager un accès jusqu'à la partie du rachis du patient sur laquelle le praticien veut intervenir, et en particulier jusqu'au disque intervertébral qu'il veut opérer, le praticien positionne sensiblement verticalement les première et deuxième parois d'écartement 120, 130 en regard, à l'état fermé, sur les deux vertèbres adjacentes 1, 2 positionnées de part et d'autre du disque intervertébral.

Dans cette position, les deux parois d'écartement 120, 130 écartent les parties molles du patient de la zone du rachis à opérer en prenant appui sur ces dernières, et sont légèrement inclinées l'une par rapport à l'autre pour former un entonnoir légèrement évasé.

Les clous de fixation 141, 142, 143, 144 sont alors engagés dans les conduits de réception correspondants 170 des panneaux desdites parois 120, 130 et enfoncés dans chaque vertèbre 1, 2.

Dans une deuxième étape, la pièce plate en forme de C 110 est positionnée sur les pions verticaux 121a, 131a des panneaux fixes 121, 131 des première et deuxième parois d'écartement 120, 130, de façon à solidariser entre eux lesdits panneaux fixes 121, 131 pour maintenir les première et les deuxième parois 120, 130 en positon fixe l'une par rapport à l'autre.

Dans une troisième étape, la troisième paroi d'écartement 150 est montée sur la pièce plate en forme de C 110 par l'intermédiaire d'une de ses fentes, 161, 162, 163 engagée dans le logement de réception correspondant 113 de ladite pièce 110. Cette troisième paroi 150 repousse également les parties molles du patient à l'extérieur de la zone intervertébrale choisie.

Comme cela a déjà été mentionné, on peut ajuster à l'aide de ses fentes, la position en hauteur de ladite troisième paroi d'écartement 150.

Chacune des parois d'écartement permet alors de retenir les parties molles du patient à l'extérieur de la zone à opérer où se trouve le disque intervertébral.

Dans une étape suivante représentée plus particulièrement sur la figure 4, les vertèbres 1 et 2 adjacentes sont écartées l'une de l'autre par le praticien au moyen d'une pince, ce qui provoque le coulissement des panneaux mobiles 122, 132 par rapport aux panneaux fixes 121, 131 des première et deuxième parois d'écartement 120, 130. On obtient ainsi une extension des première et deuxième parois d'écartement qui suivent le mouvement des vertèbres.

A l'état ouvert des première et deuxième parois d'écartement 120, 130, le chirurgien a un champ dégagé exempt de toutes parties molles retenues, pour accéder au disque intervertébral souhaité, ou plus généralement à la zone intervertébrale à opérer.

A cet effet, la courbure desdites première et deuxième parois, augmente encore le champ d'accès à ladite zone intervertébrale.

Le dispositif d'écartement selon l'invention présente plus particulièrement les avantages suivants :
- il permet d'écarter les parties molles en trois points différents dont les positions relatives sont ajustables,
- les panneaux mobiles des première et deuxième parois d'écartement permettent de suivre la distraction des vertèbres,
- il présente une profondeur modulable grâce au système montable et démontable des parois d'écartement sur la pièce plate de solidarisation, en faisant varier la longueur desdites parois d'écartement,
- les poignées amovibles permettent une mise en place et une extraction aisée du dispositif,
- il est simple, ergonomique, et son encombrement est réduit.

L'étendue de la protection pour la présente invention est définie par les revendications ci-après. Elle n'est nullement limitée aux modes de réalisation décrits nullement limitée aux modes de réalisation décrits et représentés ici.

En particulier, il est intéressant de préciser que le dispositif selon l'invention peut avantageusement être incorporé dans un ensemble comprenant en outre des instruments spécifiques chirurgicaux.

## Revendications

1. Dispositif d'écartement (100) de parties molles d'un corps humain ou d'un vertébré, pour accéder par voie antérieure à une partie du rachis dudit corps, **caractérisé en ce qu'**il comprend :
- une première et une deuxième parois d'écartement (120, 130) de parties molles, disposées en regard l'une de l'autre, et pourvues chacune d'un panneau fixe (121, 131) et d'un panneau mobile (122, 132) apte à coulisser par rapport audit panneau fixe (121, 131), sensiblement transversalement à la direction longitudinale desdites parois d'écartement (120, 130), ces dernières étant destinées à être positionnées chacune sensiblement verticalement, à cheval sur deux vertèbres adjacentes du rachis,
- des moyens de fixation (141, 142, 143, 144) de chaque panneau fixe (121, 131) sur une vertèbre et de chaque panneau mobile (122, 132) à l'autre vertèbre adjacente, et
- des moyens de solidarisation (110, 121a, 131a,) des panneaux fixes (121, 131) entre eux.

2. Dispositif d'écartement (100) selon la revendication 1, **caractérisé en ce qu'**il comprend une troisième paroi d'écartement de parties molles, comportant un panneau fixe (150) positionné dans un plan longitudinal et perpendiculaire aux plans desdites première et deuxième parois (120, 130), et des moyens de montage (160) de la troisième paroi d'écartement (150) auxdits moyens de solidarisation (110), ladite troisième paroi (150) étant destinée à être positionnée de manière rigide, sensiblement verticalement au niveau d'une des deux vertèbres supportant les première et deuxième parois d'écartement (120, 130), de sorte qu'elle fait face aux deux bords longitudinaux externes (121b, 131b) des panneaux fixes en regard (121,131) desdites première et deuxième parois d'écartement (120, 130).

3. Dispositif d'écartement (100) selon l'une des revendications 1 ou 2, **caractérisé en ce qu'**il comporte une poignée de préhension et de manoeuvre (171, 172, 173) montée de manière amovible sur chaque paroi d'écartement (120, 130, 150).

4. Dispositif d'écartement (100) selon l'une des revendications 1 à 3, **caractérisé en ce que** les première et deuxième parois d'écartement (120, 130) présentent un profil courbe et sont placées de sorte que leur face incurvée soit tournée vers les vertèbres, le coulissement des panneaux mobiles (122, 132) par rapport aux panneaux fixes (121, 131) s'effectuant suivant un arc de cercle.

5. Dispositif d'écartement (100) selon l'une des revendications 1 à 4,
**caractérisé en ce que** chaque panneau mobile (132) des première et deuxième parois d'écartement (120, 130) est pourvu de lumières (132c) allongées selon la direction transversale dudit panneau et réparties sur sa hauteur, dans lesquelles sont montés à coulissement des tétons (131c) prévus sur la face incurvée de chaque panneau fixe (131) des première et deuxième parois d'écartement, ou réciproquement.

6. Dispositif d'écartement (100) selon l'une des revendications 1 à 5, **caractérisé en ce que** les première et deuxième parois d'écartement (120, 130) sont symétriques par rapport à un axe vertical (X) passant entre les deux vertèbres adjacentes.

7. Dispositif d'écartement (100) selon l'une des revendications 1 à 6, **caractérisé en ce que** chaque bord externe longitudinal (121b, 131b, 122b, 132b) de chacun des panneaux fixes (121, 131) et mobiles (122, 132) desdites première et deuxième parois d'écartement (120, 130) comprend un conduit de réception (170) ouvert à chaque extrémité pour le passage d'un clou de fixation (141, 142, 143, 144) à une vertèbre.

8. Dispositif d'écartement (100) selon l'une de revendications 1 à 7, **caractérisé en ce que** lesdits moyens de solidarisation comprennent une pièce plate de forme en C (110), positionnée dans un plan horizontal, et pourvue à chaque extrémité d'un orifice (111) dans lequel est engagé un pion vertical solidaire (121a, 131a) solidaire du panneau fixe (121, 131) des première et deuxième parois d'écartement.

9. Dispositif d'écartement (100) selon la revendication 8, **caractérisé en ce que** la pièce plate en forme de C (110) comporte à chaque extrémité deux orifices (111, 112) adjacents.

10. Dispositif d'écartement (100) selon l'une des revendications 8 ou 9, **caractérisé en ce que** la pièce plate en forme de C (110) comprend dans sa partie centrale, un logement de réception (113) pour le montage de la troisième paroi d'écartement (150).

11. Dispositif d'écartement (100) selon la revendication 10 dans sa dépendance aux revendications 2 à 7, **caractérisé en ce que** la troisième paroi d'écartement (150) comporte en partie supérieure une pluralité de fentes horizontales (161, 162, 163), chaque fente étant apte à coopérer par engagement avec le logement de réception (113) de la pièce en forme de C (110).

12. Dispositif d'écartement (100) selon l'une des revendications 1 à 11, **caractérisé en ce qu'**il est réalisé en alliage métallique médical.

13. Dispositif d'écartement (100) selon l'une des revendications 1 à 11, **caractérisé en ce qu'**il est réalisé en matériau composite.

## Claims

1. Retractor (100) for retracting the soft tissues of a human body or of a vertebrate, for anterior access to part of the spine of said body, **characterized in that** it comprises:
- first and second soft-tissue retracting walls (120, 130) placed facing each other and each provided with a stationary panel (121, 131) and with a movable panel (122, 132) that can slide relative to said stationary panel (121, 131) approximately transversely to the longitudinal direction of said retracting walls (120, 130), each retracting wall being intended to be positioned approximately vertically, astride two adjacent vertebrae of the spine;
- means (141, 142, 143, 144) for fastening each stationary panel (121, 131) to one vertebra and for fastening each movable panel (122, 132) to the adjacent other vertebra, and
- means (110, 121a, 131a) for securing the stationary panels (121, 131) together.

2. Retractor (100) according to Claim 1, **characterized in that** it includes a third soft-tissue retracting wall, comprising a stationary panel (150) positioned in a longitudinal plane perpendicular to the planes of said first and second walls (120, 130), and means (160) for mounting the third retracting wall (150) on said securing means (110), said third wall (150) being intended to be rigidly positioned approximately vertically at one of the two vertebrae supporting the first and second retracting walls (120, 130) in such a way that it faces the two external longitudinal edges (121b, 131b) of the facing stationary panels (121, 131) of said first and second retracting walls (120, 130).

3. Retractor (100) according to either of Claims 1 and 2, **characterized in that** it includes a grasping and manoeuvring handle (171, 172, 173) removably mounted on each retracting wall (120, 130, 150).

4. Retractor (100) according to one of Claims 1 to 3, **characterized in that** the first and second retracting walls (120, 130) have a curved profile and are placed in such a way that their curved face is turned towards the vertebrae, the movable panels (122, 132) sliding relative to the stationary panels (121, 131) along a circular arc.

5. Retractor (100) according to one of Claims 1 to 4, **characterized in that** each movable panel (132) of the first and second retracting walls (120, 130) is provided with slots (132c) elongate in the transverse direction of said panel and distributed over its height, in which slots there are slidably mounted studs (131c) provided on the curved face of each stationary panel (131) of the first and second retracting walls, or vice versa.

6. Retractor (100) according to one of Claims 1 to 5, **characterized in that** the first and second retracting walls (120, 130) are symmetrical with respect to a vertical axis (X) passing between the two adjacent vertebrae.

7. Retractor (100) according to one of Claims 1 to 6, **characterized in that** each longitudinal external edge (121b, 131b, 122b, 132b) of each of the stationary (121, 131) and movable (122, 132) panels of said first and second retracting walls (120, 130) comprises a receiving duct (170) open at each end for passage of a nail (141, 142, 143, 144) for fastening to a vertebra.

8. Retractor (100) according to one of Claims 1 to 7, **characterized in that** said securing means comprise a flat C-shaped piece (110) that is positioned in a horizontal plane and provided at each end with an orifice (111) in which a vertical pin (121a, 131a) securely attached to the stationary panel (121, 131) of the first and second retracting walls is engaged.

9. Retractor (100) according to Claim 8, **characterized in that** the flat C-shaped piece (110) includes two adjacent orifices (111, 112) at each end.

10. Retractor (100) according to either of Claims 8 and 9, **characterized in that** the flat C-shaped piece (110) includes, in its central part, a receiving housing (113) into which the third retracting wall (150) is mounted.

11. Retractor (100) according to Claim 10 in its dependence on Claims 2 to 7, **characterized in that** the third retracting wall (150) includes, in its upper part, a plurality of horizontal slits (161, 162, 163), each slit being capable of cooperating by engagement with the receiving housing (113) of the C-shaped piece (110).

12. Retractor (100) according to one of Claims 1 to 11, **characterized in that** it is made of a medical metal alloy.

13. Retractor (100) according to one of Claims 1 to 11, **characterized in that** it is made of a composite.

## Patentansprüche

1. Aufweitungsvorrichtung (100) für weiche Teile eines menschlichen Körpers oder Wirbeltieres, um über einen vorderen Weg auf einen Teil der Wirbelsäule des Körpers zuzugreifen, **dadurch gekennzeichnet, dass** diese umfasst:
- eine erste und eine zweite Aufweitungswand (120, 130) für Weichteile, die einander gegenüber angeordnet sind und jeweils aufweisen eine feststehende Platte (121, 131) und eine bewegliche Platte (122, 132), die so ausgebildet ist, dass sie sich in Bezug zu der feststehenden Platte (121, 131) im Wesentlichen quer zur Längsrichtung der Aufweitungswände (120, 130) verschiebt, wobei diese dazu bestimmt sind, jeweils im Wesentlichen vertikal, auf zwei benachbarten Wirbeln der Wirbelsäule sitzend positioniert zu werden,
- eine Fixiereinrichtung (142, 143, 144) für jede fest stehende Platte (121, 131) auf einem Wirbel und für jede bewegliche Platte (122, 132) auf dem anderen benachbarten Wirbel, und
- eine Einrichtung zur Befestigung (110, 121 a, 131 a) der fest stehenden Platten (121, 131) untereinander.

2. Aufweitungsvorrichtung (100) nach Anspruch 1, **dadurch gekennzeichnet, dass** diese eine dritte Aufweitungswand für Weichteile umfasst, mit einer fest stehenden Platte (150), die in einer Längsebene und senkrecht zu den Ebenen der ersten und zweiten Wand (120, 130) positioniert ist, und einer Einrichtung zur Montage (160) der dritten Aufweitungswand (150) an der Befestigungseinrichtung (110), wobei die dritte Wand (150) dazu bestimmt ist, starr im Wesentlichen vertikal an einem der beiden Wirbel positioniert zu werden, welche die erste und die zweite Aufweitungswand (120, 130) abstützen, derart, dass sie den beiden äußeren Längskanten (121b, 131 b) der gegenüber liegenden fest stehenden Platten (121, 131) der ersten und zweite Aufweitungswand (120, 130) zugewandt ist.

3. Aufweitungsvorrichtung (100) nach einem der Ansprüche 1 oder 2, **dadurch gekennzeichnet, dass** diese einen Griff zum Anfassen und Handhaben (171, 172, 173) aufweist, der lösbar auf jeder Aufweitungswand (120, 130, 150) montiert ist.

4. Aufweitungsvorrichtung (100) nach einem der Ansprüche 1 bis 3, **dadurch gekennzeichnet, dass** die erste und zweite Aufweitungswand (120, 130) ein gekrümmtes Profil aufweisen und derart angeordnet sind, dass ihre einwärts gekrümmte Fläche zu den Wirbeln gerichtet ist, wobei die Verschiebung der beweglichen Platten (122, 132) gegenüber den fest stehenden Platten (121, 131) in einem Kreisbogen erfolgt.

5. Aufweitungsvorrichtung (100) nach einem der Ansprüche 1 bis 4, **dadurch gekennzeichnet, dass** jede bewegliche Platte (132) der ersten und zweiten Aufweitungswand (120, 130) mit Löchern (132c) versehen ist, die in einer Richtung quer zu der Platte länglich ausgebildet sind und über ihre Höhe verteilt sind, in welchen Ansätze (131 c) verschiebbar montiert sind, die auf der nach innen gekrümmten Fläche jeder fest stehenden Platte (131) der ersten und zweiten Aufweitungswand oder umgekehrt vorgesehen sind.

6. Aufweitungsvorrichtung (100) nach einem der Ansprüche 1 bis 5, **dadurch gekennzeichnet, dass** die erste und zweite Aufweitungswand (120, 130) symmetrisch in Bezug zu einer vertikalen Achse (X) durch die zwei benachbarten Wirbel sind.

7. Aufweitungsvorrichtung (100) nach einem der Ansprüche 1 bis 6, **dadurch gekennzeichnet, dass** jede äußere Längskante (121b, 131b, 122b, 132b) jeder der fest stehenden (121, 131) und beweglichen (122, 132) Platten der ersten und zweiten Aufweitungswand (120, 130) einen Aufnahmekanal (170) umfasst, der für den Durchgang eines Fixierstiftes (141, 142, 143, 144) an einem Wirbel an jedem Ende offen ist.

8. Aufweitungsvorrichtung (100) nach einem der Ansprüche 1 bis 7, **dadurch gekennzeichnet, dass** die Befestigungseinrichtung ein flaches C-förmiges Teil (110) umfasst, das in einer horizontalen Ebene positioniert ist und an jedem Ende mit einer Öffnung (111) versehen ist, in welche ein vertikales Metallstück (121a, 131 a) eingreift, das fest mit der fest stehenden Platte (121, 131) der ersten und zweiten Aufweitungswand verbunden ist.

9. Aufweitungsvorrichtung (100) nach Anspruch 8, **dadurch gekennzeichnet, dass** das flache C-förmige Teil (110) an jedem Ende zwei benachbarte Öffnungen (111, 112) aufweist.

10. Aufweitungsvorrichtung (100) nach einem der Ansprüche 8 oder 9, **dadurch gekennzeichnet, dass** das flache C-förmige Teil (110) in seinem Zentralbereich eine Aufnahme (113) für die Montage der dritten Aufweitungswand (150) aufweist.

11. Aufweitungsvorrichtung (100) nach Anspruch 10 in seiner Abhängigkeit zu den Ansprüchen 2 bis 7, **dadurch gekennzeichnet, dass** die dritte Aufweitungswand (150) in einem oberen Bereich eine Mehrzahl von horizontalen Schlitzen (161, 162, 163) aufweist, wobei jeder Schlitz so ausgebildet ist, dass dieser durch Eingriff in die Aufnahme (113) des C-förmigen Teils (110) zusammenwirkt.

12. Aufweitungsvorrichtung (100) nach einem der Ansprüche 1 bis 11, **dadurch gekennzeichnet, dass** diese aus einer medizinischen Metalllegierung hergestellt ist.

13. Aufweitungsvorrichtung (100) nach einem der Ansprüche 1 bis 11, **dadurch gekennzeichnet, dass** diese aus einem Verbundmaterial hergestellt ist.
